# FASCICULE DE BREVET EUROPEEN

(11) **EP 1 467 750 B1**
(45) Date de publication et mention de la délivrance du brevet: **12.07.2006**
(21) Numéro de dépôt: 03731736.9
(22) Date de dépôt: 22.01.2003
(51) Int. Cl.: A61K 31/404, A61K 9/20

(54) **COMPOSITION PHARMACEUTIQUE ORODISPERSIBLE DE PERINDOPRIL**
PERINDOPRIL ENTHALTENDE PHARMAZEUTISCHE ZUSAMMENSETZUNG MIT ORALER DISPERSION
ORODISPERSIBLE PHARMACEUTICAL COMPOSITION COMPRISING PERINDOPRIL

(30) Priorité: 23.01.2002 FR 0200790
(43) Date de publication de la demande: 20.10.2004
(73) Titulaire: Les Laboratoires Servier, 92415 Courbevoie Cédex (FR)
(72) Inventeur: WUTHRICH, Patrick, F-45000 Orleans (FR); ROLLAND, Hervé, F-45160 Olivet (FR); JULIEN, Marc, F-45110 Sigloy (FR)
(86) Numéro de dépôt international: PCT/FR2003/000200
(87) Numéro de publication internationale: WO 2003/061691

(56) Documents cités:
- EP-A- 0 192 080
- EP-A- 0 745 382
- EP-A- 1 175 899
- EP-A- 1 203 580
- WO-A-01/83439

## Description

La présente invention a pour objet une forme pharmaceutique orodispersible solide pour l'administration par voie orale de périndopril, ou de ses sels pharmaceutiquement acceptables, sans prise simultanée d'un verre d'eau et sans problème de déglutition.

Le périndopril est un composé antihypertenseur qui exerce notamment une activité inhibitrice sur certaines enzymes, comme les carboxypolypeptidases, les enképhalinases ou la kininase II. Il inhibe notamment la transformation du décapeptide angiotensine I en l'octapeptide angiotensine II, responsable dans certains cas de l'hypertension artérielle, en agissant sur l'enzyme de conversion.

L'emploi en thérapeutique du Périndopril et de ses sels pharmaceutiquement acceptables permet, de réduire ou même supprimer l'activité de ces enzymes responsables de la maladie hypertensive ou de l'insuffisance cardiaque. L'action sur la kininase II a pour résultat l'augmentation de la bradykinine circulante et également la baisse de la tension artérielle par cette voie.

Actuellement, le sel de *tert*-butylamine du périndopril est administré par voie orale sous forme de comprimés à avaler avec un demi-verre d'eau. Ces comprimés de périndopril sont utiles pour le traitement de l'hypertension artérielle et l'insuffisance cardiaque congestive.

Les doses de sel de *tert*-butylamine du périndopril couramment prescrites vont de 1 mg à 8 mg par jour sous la forme de comprimé à libération immédiate.

De nombreuses personnes ont des difficultés pour avaler les comprimés conventionnels souvent de taille non négligeable, comme les enfants et les personnes âgées. Les problèmes liés à l'ingestion de médicaments (étouffement, fausse route, suffocation par obstruction de la gorge) sont souvent à l'origine d'un mauvais respect des posologies, voire d'un arrêt du traitement.

Les compositions pharmaceutiques de la présente invention permettent non seulement de remédier aux inconvénients connus de la forme comprimé à avaler mais également de proposer un service médical rendu supérieur permettant notamment l'amélioration de la qualité de vie des patients.

La composition pharmaceutique orodispersible de périndopril présente l'avantage d'une obtention rapide de taux plasmatiques élevés en principe actif

La composition pharmaceutique orodispersible selon l'invention présente la particularité de ne nécessiter ni eau ni mastication au cours de son administration. Elle se désagrège très rapidement dans la bouche, de préférence en moins de trois minutes et de manière encore plus préférentielle en moins d'une minute.

De nombreuses formes à dissolution rapide sont décrites dans l'art antérieur. De manière générale, les technologies décrites précédemment ont en commun l'utilisation d'un agent désintégrant comme le Kollidon® CL (polyvinylpyrrolidone réticulée), l'EXPLOTAB® (fécule carboxyméthylée), l'AC DISOL® (carboxyméthylcellulose sodique réticulée).

Cet agent de désintégration est indispensable dans la formulation des comprimés orodispersibles et doit être utilisé conjointement avec un excipient de compression directe. Les difficultés rencontrées pour la fabrication de tels comprimés résident dans le fait qu'il est très difficile d'obtenir des comprimés présentant des caractéristiques physiques constantes et reproductibles et compatibles avec les contraintes de manipulation classiques des comprimés.

En effet, les mélanges classiquement utilisés conduisent à des comprimés de dureté très élevée totalement inadaptée à une désagrégation rapide dans la cavité buccale.

D'autres formes orodispersibles sont réalisables par l'utilisation de la lyophilisation aboutissant à l'obtention de formes solides très poreuses dénommées "lyophilisat oral".

Ces formes nécessitent l'utilisation d'un procédé industriel très spécifique, compliqué et long de mise en oeuvre, donnant une forme médicamenteuse à prix de revient élevé. De plus, le procédé de fabrication par lyophilisation nécessite une étape de dissolution dans l'eau du principe actif pouvant entraîner une dégradation de celui-ci.

La présente invention permet de remédier à ces inconvénients. Elle concerne une forme solide orodispersible de périndopril contenant un excipient simple, d'origine naturelle permettant la désagrégation rapide, présentant une neutralité gustative et de texture agréable. Cet excipient joue le rôle à la fois de liant et de désintégrant. Il permet d'obtenir une formulation de périndopril simple, sans utilisation d'eau dans le procédé de fabrication, ayant une excellente aptitude à la compression directe conduisant à des comprimés de faible friabilité et de dureté compatible avec les techniques classiques de manipulation.

D'autre part, cet excipient permet d'obtenir des comprimés de très petite taille qui peuvent être administrés à des enfants en très bas âge. En effet, pour les comprimés pédiatriques, il est nécessaire que la prise soit facilitée, que la désagrégation soit très rapide en bouche afin d'éviter que l'enfant ne rejette la forme et que les comprimés aient une dureté suffisante pour une manipulation aisée et un conditionnement simple (blister, distributeur unitaire adapté).

Les formes orodispersibles selon l'invention permettent de réaliser des comprimés pédiatriques de très faible taille (diamètre 3 mm, épaisseur à partir de 1 mm et masse à partir de 10 mg), facilement manipulables et se désagrégeant en quelques secondes en bouche.

Plus particulièrement, l'invention concerne une composition pharmaceutique solide orodispersible de périndopril caractérisée en ce qu'elle contient :
- du périndopril ou un de ses sels pharmaceutiquement acceptables,
- et des granules consistant en lactose et amidon coséchés.

La composition selon l'invention peut également contenir, pour des raisons de fabrication des composés, un ou plusieurs lubrifiants et un agent d'écoulement ainsi que des arômes, des colorants et des édulcorants, classiquement utilisés.

Dans les compositions pharmaceutiques selon l'invention, le périndopril se trouve préférentiellement sous la forme de sel de *tert*-butylamine.

L'invention a également pour objet l'utilisation de granules consistant en lactose et amidon coséchés pour la préparation de compositions pharmaceutiques solides orodispersibles de périndopril.

On entend par le terme "orodispersible" des compositions pharmaceutiques solides qui se délitent dans la cavité buccale en moins de 3 minutes, et de préférence en moins d'une minute.

Lesdits granules compris dans les compositions pharmaceutiques solides selon l'invention correspondent aux compositions décrites dans la demande de brevet EP 00/402159.8 (EP-A1-1175899). Ces granules sont caractérisés par une structure sphérique et une comprimabilité avantageuse et sont commercialisés sous l'appellation STARLAC® .

Les propriétés désintégrantes desdits granules sont connues pour des comprimés placés dans des volumes de liquides importants, sous agitation. Il est particulièrement surprenant que de tels granules employés pour la fabrication de formes orodispersibles puissent donner des résultats particulièrement satisfaisants en terme de désagrégation en bouche, et ce pour deux raisons.

La première est basée sur le constat que les excipients les moins solubles dans l'eau sont les plus appropriés à la formulation de comprimés orodispersibles (la solubilistion, entraînant une augmentation de viscosité de l'eau, est un frein à sa pénétration dans les comprimés). Or lesdits granules comprennent une fraction importante de lactose très soluble dans l'eau. De plus, l'amidon compris dans lesdits granules n'est pas un agent "super désintégrant" tel qu'utilisé et décrit dans les formes orodispersibles de l'art antérieur.

La deuxième est basée sur le constat que les propriétés de désintégration d'un excipient (utilisé dans un comprimé) évaluées dans l'eau par les méthodes conventionnelles ne sont pas extrapolables au comportement du même comprimé in vivo, dans la salive. En effet, les vitesses de désintégration dans l'eau sont mesurées (selon la Pharmacopée Européenne) dans une quantité d'eau suffisamment importante pour ne pas atteindre la saturation en terme de solubilisation, alors que in vivo, de par le faible volume de salive, les excipients sont à saturation. De plus, l'agitation à laquelle sont soumis les comprimés lors du test usuel ne reflète pas la désagrégation en bouche. La Demanderesse a ainsi constaté lors d'essais comparatifs que certains excipients connus comme bons désintégrants n'étaient pas adaptés à la préparation de formes orodispersibles. Inversement, certains excipients se désintégrant moyennement dans l'eau peuvent présenter des propriétés avantageuses in vivo.

La Demanderesse a alors trouvé que lesdits granules conféraient de façon surprenante aux comprimés de très bonnes aptitudes à se désagréger en bouche, et ce pour une large gamme de duretés de comprimés, tout en conservant une friabilité faible ce qui est particulièrement remarquable. En effet, la plupart des formes orodispersibles de l'art antérieur qui se délitent rapidement dans la bouche sont très friables, ce qui se traduit par la nécessité d'utiliser un conditionnement spécifique et par des risques de désagrégation du comprimé dès qu'il est manipulé et ôté de son emballage.

Il est particulièrement remarquable que les critères d'orodispersibilité et de friabilité faible précités soient respectés pour une large gamme de dureté de comprimés, c'est-à-dire pour des comprimés présentant une dureté comprise entre 5 et 50 Newtons (préférentiellement de 10 à 20 Newtons).

Les compositions pharmaceutiques selon l'invention sont préférentiellement caractérisées en ce qu'elles contiennent, par rapport au poids total du comprimé :
- de 0,1 % à 10 % en poids de périndopril ou d'un de ses sels pharmaceutiquement acceptables et préférentiellement de 0,5 % à 6 %,
- de 85 % à 99 % en poids de STARLAC® .

Elles contiendront éventuellement de 0,1 % à 3 % en poids d'agents lubrifiants comme le stéaryl-fumarate de sodium ou le stéarate de magnésium (préférentiellement de 0,5 % à 1,5 %), de 0,1 % à 3 % en poids d'un agent d'écoulement comme la silice colloïdale (préférentiellement de 0,5 % à 1,5 %) et de 0,1 % à 1 % en poids d'un agent édulcorant comme l'aspartame et/ou l'acesulfame K (préférentiellement de 0,2 % à 0,5 %).

Les exemples suivants illustrent l'invention mais ne la limitent en aucune façon :

Comprimés orodispersibles de périndopril

### EXEMPLE 1 :

### Formulation : Comprimé terminé à 100 mg

| ***Constituants*** | ***Quantité (mg)*** |
|---|---|
| Périndopril *tert*-butylamine | 4 |
| Starlac® | 94 |
| Stearyl-fumarate de sodium | 1,5 |
| Silice colloïdale anhydre | 0,5 |

### EXEMPLE 2 :

### Formulation : Comprimé terminé à 200 mg

| ***Constituants*** | ***Quantité (mg)*** |
|---|---|
| Périndopril *tert*-butylamine | 8 |
| Starlac® | 188 |
| Stearyl-fumarate de sodium | 3 |
| Silice colloïdale anhydre | 1 |

Les comprimés sont préparés par mélange des constituants suivi d'une compression directe. La dureté des comprimés des exemples 1 et 2 est environ égale à 20 Newtons.

Afin d'évaluer le temps de désagrégation en bouche, les comprimés orodispersibles de périndopril décrits dans les exemples 1 et 2 ont été placés dans la bouche. Lors de ces tests, il s'est avéré que pour chacune des formulations testées le temps de désagrégation dans la bouche était inférieur à 1 minute.

### EXEMPLE 3 :

### Formulation : Comprimé terminé à 10 mg

| ***Constituants*** | ***Quantité (mg)*** |
|---|---|
| Périndopril tert-butylamine | 0.0625 |
| Starlac® | 9.8375 |
| Magnesium stearate MF3 | 0.05 |
| Silice colloïdale anhydre (Aerosil 200) | 0.05 |

Comprimés de dureté de 5 à 10 newtons.

### EXEMPLE 4 :

### Formulation : Comprimé terminé à 20 mg

| ***Constituants*** | ***Quantité* (*mg*)** |
|---|---|
| Périndopril *tert*-butylamine | 0.125 |
| Starlac® | 19.675 |
| Magnesium stearate MF3 | 0.1 |
| Silice colloïdale anhydre (Aerosil 200) | 0.1 |

Comprimés de dureté de 10 à 15 newtons.

### EXEMPLE 5 :

### Formulation : Comprimé terminé à 20 mg

| ***Constituants*** | ***Quantité (mg)*** |
|---|---|
| Périndopril *tert*-butylamine | 0.25 |
| Starlac® | 19.55 |
| Magnesium stearate MF3 | 0.1 |
| Silice colloïdale anhydre (Aerosil 200) | 0.1 |

Comprimés de dureté de 10 à 15 newtons.

### EXEMPLE 6 :

### Formulation : Comprimé terminé à 20 mg

| ***Constituants*** | ***Quantité (mg)*** |
|---|---|
| Périndopril *tert*-butylamine | 1 |
| Starlac® | 18.8 |
| Magnesium stearate MF3 | 0.1 |
| Silice colloïdale anhydre (Aerosil 200) | 0.1 |

Comprimés de dureté de 10 à 15 newtons.

### EXEMPLE 7 :

### Formulation : Comprimé terminé à 20 mg avec édulcorants)

| ***Constituants*** | ***Quantité* (*mg*)** |
|---|---|
| Périndopril tert-butylamine | 1 |
| Starlac® | 18.76 |
| **Acesulfame K** | 0.02 |
| **Aspartam** | 0.02 |
| Magnesium stearate MF3 | 0.1 |
| Silice colloïdale anhydre (Aerosil 200) | 0.1 |

Comprimés de dureté de 10 à 15 newtons.

## Revendications

1. Composition pharmaceutique solide orodispersible de périndopril, ou de ses sels pharmaceutiquement acceptables, **caractérisée en ce qu'**elle comprend :
- du périndopril ou un de ses sels pharmaceutiquement acceptables,
- des granules consistant en lactose et amidon coséchés.

2. Composition pharmaceutique selon la revendication 1 **caractérisée en ce qu'**elle comprend, par rapport au poids total de la composition :
- de 0,1 % à 10 % en poids de périndopril ou d'un de ses sels pharmaceutiquement acceptables,
- de 85 % à 99 % en poids de granules consistant en lactose et amidon coséchés.

3. Composition pharmaceutique selon la revendication 2 **caractérisée en ce qu'**elle comprend de 0,5 % à 6 % en poids de périndopril, ou d'un de ses sels pharmaceutiquement acceptables.

4. Composition pharmaceutique selon la revendication 1 **caractérisée en ce qu'**elle comprend également un ou plusieurs lubrifiants, un agent d'écoulement et éventuellement une agent édulcorant.

5. Composition pharmaceutique selon la revendication 1 **caractérisée en ce qu'**elle se présente sous forme de comprimé.

6. Comprimé selon la revendication 5 **caractérisé en ce qu'**il est obtenu par compression directe.

7. Comprimé selon la revendication 6 **caractérisé en ce que** sa dureté est comprise entre 5 et 50 Newtons.

8. Comprimé selon la revendication 7 **caractérisé en ce que** sa dureté est comprise entre 10 et 20 Newtons.

9. Utilisation de granules consistant en lactose et amidon coséchés dans la fabrication des compositions solides orodispersibles de périndopril, ou de ses sels pharmaceutiquement acceptables, se délitant en bouche en moins de trois minutes et de préférence en moins d'une minute.

10. Composition pharmaceutique solide orodispersible de périndopril ou d'un de ses sels pharmaceutiquement acceptables, selon la revendication 1, utile pour le traitement de l'hypertension artérielle et l'insuffisance cardiaque.

## Claims

1. Solid orodispersible pharmaceutical composition of perindopril, or pharmaceutically acceptable salts thereof, **characterised in that** it comprises:
- perindopril or a pharmaceutically acceptable salt thereof,
- granules consisting of co-dried lactose and starch.

2. Pharmaceutical composition according to claim 1, **characterised in that** it comprises, in relation to the total weight of the composition :
- from 0.1 % to 10 % by weight of perindopril or a pharmaceutically acceptable salt thereof,
- from 85 % to 99 % by weight of granules consisting of co-dried lactose and starch.

3. Pharmaceutical composition according to claim 2, **characterised in that** it comprises from 0.5 % to 6 % by weight of perindopril or a pharmaceutically acceptable salt thereof.

4. Pharmaceutical composition according to claim 1, **characterised in that** it also comprises one or more lubricants, a flow agent and, optionally, a sweetening agent.

5. Pharmaceutical composition according to claim 1, **characterised in that** it is in the form of a tablet.

6. Tablet according to claim 5, **characterised in that** it is obtained by direct compression.

7. Tablet according to claim 6, **characterised in that** its hardness is from 5 to 50 Newtons.

8. Tablet according to claim 7, **characterised in that** its hardness is from 10 to 20 Newtons.

9. Use of granules consisting of co-dried lactose and starch in the manufacture of solid orodispersible compositions of perindopril, or pharmaceutically acceptable salts thereof, which disintegrate in the mouth in less than three minutes, preferably less than one minute.

10. Solid orodispersible pharmaceutical composition of perindopril or a pharmaceutically acceptable salt thereof, according to claim 1, for use in the treatment of arterial hypertension and heart failure.

## Patentansprüche

1. Feste, oral dispergierbare pharmazeutische Zubereitung von Perindopril oder seinen pharmazeutisch annehmbaren Salzen, **dadurch gekennzeichnet, daß** sie:
- Perindopril oder eines seiner pharmazeutisch annehmbaren Salze und
- aus gemeinsam getrockneter Lactose und Stärke bestehende Körnchen umfaßt.

2. Pharmazeutische Zubereitung nach Anspruch 1, **dadurch gekennzeichnet, daß** sie, bezogen auf das Gesamtgewicht der Zubereitung:
- 0,1 bis 10 Gew.-% Perindopril oder eines seiner pharmazeutisch annehmbaren Salze und
- 85 bis 99 Gew.-% aus gemeinsam getrockneter Lactose und Stärke bestehende Körnchen umfaßt.

3. Pharmazeutische Zubereitung nach Anspruch 2, **dadurch gekennzeichnet, daß** sie 0,5 bis 6 Gew.-% Perindopril oder eines seiner pharmazeutisch annehmbaren Salze umfaßt.

4. Pharmazeutische Zubereitung nach Anspruch 1, **dadurch gekennzeichnet, daß** sie zusätzlich ein oder mehrere Gleitmittel, ein Fließmittel und gegebenenfalls ein Süßungsmittel umfaßt.

5. Pharmazeutische Zubereitung nach Anspruch 1, **dadurch gekennzeichnet, daß** sie in Form einer Tablette vorliegt.

6. Tablette nach Anspruch 5, **dadurch gekennzeichnet, daß** sie durch direktes Verpressen gebildet worden ist.

7. Tablette nach Anspruch 6, **dadurch gekennzeichnet, daß** ihre Härte zwischen 5 und 50 Newton liegt.

8. Tablette nach Anspruch 7, **dadurch gekennzeichnet, daß** ihre Härte zwischen 10 und 20 Newton liegt.

9. Verwendung von aus gemeinsam getrockneter Lactose und Stärke bestehenden Körnchen für die Herstellung von festen, oral dispergierbaren Zubereitungen von Perindopril oder einem seiner pharmazeutisch annehmbaren Salze, die in weniger als drei Minuten, vorzugsweise weniger als einer Minute, im Mund zerfallen.

10. Feste, oral dispergierbare pharmazeutische Zubereitung von Perindopril oder einem seiner pharmazeutisch annehmbaren Salze nach Anspruch 1, nützlich zur Behandlung von arterieller Hypertension und von Herzinsuffizienz.
